(19) Europäisches Patentamt
European Patent Office
Office européen des brevets

(11) **EP 3 643 316 A1**

(12) **EUROPEAN PATENT APPLICATION**
published in accordance with Art. 153(4) EPC

(43) Date of publication:
**29.04.2020 Bulletin 2020/18**

(51) Int Cl.:
*A61K 35/545* (2015.01)     *A61K 35/28* (2015.01)
*A61K 35/33* (2015.01)     *A61K 35/36* (2015.01)
*A61P 17/00* (2006.01)     *A61P 17/02* (2006.01)
*C12N 5/071* (2010.01)     *C12N 5/0775* (2010.01)

(21) Application number: 18820712.0

(22) Date of filing: 19.06.2018

(86) International application number:
**PCT/JP2018/023352**

(87) International publication number:
**WO 2018/235834 (27.12.2018 Gazette 2018/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.06.2017 JP 2017119854**

(71) Applicants:
• **National University Corporation
Hokkaido University
Sapporo-shi, Hokkaido 060-0808 (JP)**

• **Life Science Institute, Inc.
Tokyo 100-8251 (JP)**

(72) Inventors:
• **SHIMIZU, Hiroshi
Sapporo-shi
Hokkaido 060-0808 (JP)**
• **FUJITA, Yasuyuki
Sapporo-shi
Hokkaido 060-0808 (JP)**
• **MASUTOMI, Naoya
Tokyo 101-0047 (JP)**

(74) Representative: **Gill Jennings & Every LLP
The Broadgate Tower
20 Primrose Street
London EC2A 2ES (GB)**

(54) **TREATMENT AGENT FOR EPIDERMOLYSIS BULLOSA**

(57)     A cell product for treatment of epidermolysis bullosa, comprising a SSEA-3-positive pluripotent stem cell (Muse cell) derived from a mesenchymal tissue in a living body or a cultured mesenchymal cell. Preferably, the epidermolysis bullosa is epidermolysis bullosa simplex, junctional epidermolysis bullosa, or dystrophic epidermolysis bullosa.

Fig. 1

**Description**

TECHNICAL FIELD

The present invention relates to a cell product in regenerative therapy. More particularly, the present invention relates to a cell product comprising a pluripotent stem cell, the cell product being effective for treatment of epidermolysis bullosa, and to a cell product comprising a skin cell differentiated from a pluripotent stem cell, the cell product being effective for treatment of skin diseases such as epidermolysis bullosa.

BACKGROUND ART

Epidermolysis bullosa (EB) is a serious hereditary bullous skin disease in which genetic abnormalities in adhesion structure control proteins in a skin basement membrane zone disrupt the adhesion functions between epidermis and dermis, allowing epidermis to peel off at the basement membrane level with a slight external force in daily life and forming blisters and/or ulcers (Table 1). Depending on the site where blisters form, epidermolysis bullosa is divided into three main types: epidermolysis bullosa simplex, junctional epidermolysis bullosa, and dystrophic epidermolysis bullosa. Epidermolysis bullosa causes blisters and/or erosions with slight external forces at sites that are susceptible to external forces, such as peripheral extremities and large joints. Blisters and erosions associated with epidermolysis bullosa simplex and dominant epidermolysis bullosa dystrophica are relatively rapidly healed. After being healed, they do not leave scars or skin atrophy in epidermolysis bullosa simplex but leave scars in dominant epidermolysis bullosa dystrophica. On the other hand, blisters and erosions associated with junctional epidermolysis bullosa and recessive epidermolysis bullosa dystrophica are intractable in general. When healed, they leave skin atrophy in junctional epidermolysis bullosa while leaving scars in recessive epidermolysis bullosa dystrophica. Epidermolysis bullosa is difficult to identify from clinical findings at birth, and thus comprehensive diagnosis is made in combination with electron microscope, immunostaining, genetic diagnosis, and clinical findings changing with growth. Early diagnosis provides effective information to successfully manage skin and whole body. Diagnosis and treatment of rare intractable diseases require special facilities as well as examination by and advice from medical specialist.

Table 1

| <Disease types, Responsible Genes and Proteins> | | |
|---|---|---|
| Type | Main Subtype | Responsible Genes and Proteins |
| Epidermolysis Bullosa Simplex | Weber-Cockayne | Keratin 5, Keratin 14 |
| | Koebner | |
| | Dowling-Meara | |
| | with muscular dystrophy | Plectin |
| | with pyloric atresia | Plectin |
| Junctional Epidermolysis Bullosa | Herlitz | LAMA3. LAMB3, LAMC2(Laminin 332) |
| | non- Herlitz | LAMA3, LAMB3, LAMC2(Laminin 332) |
| | | Collagen type XVII |
| | with pyloric atresia | ITGA6. ITGB4( $\alpha$ 6 $\beta$ 4 Integrin) |
| Dystrophic Epidermolysis Bullosa | Dominant | Collagen type VII |
| | Severe generalized recessive | |
| | Recessive, other generalized | |
| Cited from homepage for research groups on rare intractable skin diseases "epidermolysis bullosa" | | |

Methods for treating epidermolysis bullosa are symptomatic treatments only, and radical treatment does not exist currently. Since the symptomatic treatments also depend on the type of the disease, accurate diagnosis of type is first essential. In addition, epidermolysis bullosa may develop various complications depending on the type and worsen the condition, significantly restricting daily living of the patient, and thus treatments for the various complication are also needed. Furthermore, since epidermolysis bullosa is an intractable hereditary disease, it is also necessary to consider prevention of recurrence in patients in the family.

<Local Treatment>

A blister, erosion, or ulcer is washed with water, and then a petrolatum gauze or the like is applied thereon to prevent adhesion between the gauze and the erosion. At this time, the blister is pre-punctured to remove the content (without removing the blister lid). In cases involving adhesion between fingers, a petrolatum gauze or the like is put between the fingers to prevent adhesion between fingers. Because prolonged use of ointments containing antibiotics can cause the emergence of resistant bacteria, ointments containing antibiotics are not positively and necessarily used except in special cases. If erosion or ulcer worsens, there can be risk of complicated fungal or bacterial infections, and skin carcinoma particularly in recessive dystrophic epidermolysis bullosa. For this reason, skin biopsy, fungal test, bacterial culture test, and the like are positively performed. In general, ointment treatment is applied once a day.

<Systemic Treatment>

Nutrition Supplementation: Especially in cases of recessive dystrophic epidermolysis bullosa, due to oral mucosal and esophageal lesions, nutrient intake is insufficient, and thus chronic malnutrition and anemia are very common. Therefore, oral intake of nutrients such as ENSURE LIQUID is useful. If oral intake is difficult, nutrients may be supplemented with a nasal tube or infusion. Anti-histamines may be effectively used for severe itching.

<Treatment for Complications>

In cases of recessive epidermolysis bullosa dystrophica and junctional epidermolysis bullosa, for example, adhesion between fingers, malignant skin tumor, esophageal stenosis, pyloric stenosis, anal erosion and stenosis, malnutrition, conjunctival erosion, anemia are often problematic. In addition, one of serious complications is secondary systemic amyloidosis.

Many of the complications of epidermolysis bullosa significantly reduce the quality of life, and therefore the demand for therapy is high. It is important to provide diagnosis and treatment with the cooperation of specialists in various clinical fields, such as plastic surgery, dilation, and nutrition management (Non-Patent Document 1).

However, since a safe and reliable methodology for normalizing genetic abnormalities has not yet been established, there is no radical treatment for hereditary diseases such as epidermolysis bullosa at present.

On the other hand, with the recent progress in research on regenerative therapy, treatments of epidermolysis bullosa by bone marrow transplantation, bone marrow stem cell transplantation and the like have been searched.

For example, findings as described below have been reported (Non-Patent Document 2).

(1) Mechanism of Skin Regeneration by Bone Marrow-derived Cells: As a mechanism of epidermal regeneration in skin of a patient with epidermolysis bullosa, where large amounts of epidermal stem cells have been lost after many years of extensive epidermal detachment, stem cells in the bone marrow are found to be recruited to the injured skin through the peripheral circulation and contribute to the regeneration of the skin with blisters (Non-Patent Documents 3 and 4).

(2) Bone Marrow Transplantation Therapy for Epidermolysis Bullosa: A research group at the University of Minnesota, USA, performed for the first time in the world bone marrow transplantation for recessive dystrophic epidermolysis bullosa, and reported the effect of improving the skin symptoms. However, in 2 of 7 cases patients died during the course, and thus development of a safer protocol for bone marrow transplantation therapy is essential (Non-Patent Document 5).

(3) Bone Marrow Mesenchymal Stem Cell Transplantation Therapy for Epidermolysis Bullosa: A study group in Chile, South America, subcutaneously transplanted bone marrow-derived cultured mesenchymal stem cells from a healthy subject to two cases of severe recessive dystrophic epidermolysis bullosa, and determined the effectiveness (Non-Patent Document 6). In addition, UK and Egyptian groups administered via infusion marrow-derived cultured mesenchymal stem cells from a healthy subject to patients with severe recessive dystrophic epidermolysis bullosa, and reported the effectiveness (Non-Patent Documents 7 and 8). However, it was also found that the transplanted mesenchymal stem cells can gradually decrease in a few months.

(4) Feasibility of Regeneration-inducing Therapy for Epidermolysis Bullosa Using a Factor for Recruiting Bone Marrow Mesenchymal Stem Cell in Blood: HMGB1 released from detached epidermis was found to cause bone marrow mesenchymal stem cells to accumulate in detached epidermal skin via peripheral blood and strongly induce regeneration of the damaged skin (Non-Patent Document 4).

As described above, intensive basic and clinical researches on gene therapy and regenerative therapy are progressed with the aim of developing radical therapy for epidermolysis bullosa. However, safe and effective therapy that completely cures epidermolysis bullosa has not yet been found, and therefore further radical therapies are demanded to be realized.

Studies by Izawa et al. have revealed that pluripotent stem cells that are present in mesenchymal cell fractions, can be obtained without gene introduction or induction by cytokines or the like, and express SSEA-3 (Stage-Specific Embryonic Antigen-3) as a surface antigen (Multilineage-differentiating Stress Enduring cells; Muse cell) can be responsible for the pluripotency possessed by the mesenchymal cell fractions, and applied to disease treatment aimed at tissue regeneration (e.g., Patent Document 1; Non-Patent Documents 9 to 11). However, it has not been demonstrated whether use of Muse cells in treatment of epidermolysis bullosa could provide expected therapeutic effects.

PRIOR ART DOCUMENTS

PATENT DOCUMENTS

Patent Document 1: Japanese Patent No. 5185443

NON-PATENT DOCUMENTS

Non-patent Document 1: Homepage for research groups on rare intractable skin diseases (http://kinan.info/)
Non-patent Document 2: Tamai K. J Natl Inst Public Health 2011; 60: 118-124.
Non-patent Document 3: Chino T et al. Am J Pathol 2008; 173: 803-814.
Non-patent Document 4: Tamai K et al. Proc Natl Acad Sci USA 2011; 108: 6609-6614.
Non-patent Document 5: Wagner JE et al. N Engl J Med 2010; 363: 629-639.
Non-patent Document 6: Conget P et al. Cytotherapy 2010; 12: 429-431.
Non-patent Document 7: Petrof G et al. J Invest Dermatol 2015; 135: 2319-2321.
Non-patent Document 8: El-Darouti M et al. Dermatol Ther 2016; 29: 96-100.
Non-patent Document 9: Kuroda Y et al. Proc Natl Acad Sci USA 2010; 107: 8639-8643.
Non-patent Document 10: Wakao S et al. Proc Natl Acad Sci USA 2011; 108: 9875-9880.
Non-patent Document 11: Kuroda Y et al. Nat Protc 2013; 8: 1391-1415.

SUMMARY OF THE INVENTION

An object of the present invention is to provide a cell product for treatment of skin diseases such as epidermolysis bullosa.

The present inventors found that administration of human Muse cells to a mouse having experimentally injured skin via its blood vessel leads to expression of human collagen at the wound site, which facilitates wound repair. The present inventors also found that after blister formation in the epidermis of a collagen type XVII (COL17) gene-deficient mouse model of epidermolysis bullosa, administration of human Muse cells via its blood vessel or the like can provide human collagen type XVII to the epidermis, meaning that Muse cells can be used in treatment of epidermolysis bullosa. The present inventors further found that skin cells that are effective in treatment of skin diseases, such as keratinocytes and fibroblasts, can be obtained from Muse cells, thereby completed the present invention.

Accordingly, the present invention provides the following [1] to [11]:

[1] A cell product for treatment of epidermolysis bullosa, comprising a SSEA-3-positive pluripotent stem cell derived from a mesenchymal tissue in a living body or a cultured mesenchymal cell;
[2] The cell product of item [1], wherein said epidermolysis bullosa is epidermolysis bullosa simplex;
[3] The cell product of item [1], wherein said epidermolysis bullosa is junctional epidermolysis bullosa;
[4] The cell product of item [1], wherein said epidermolysis bullosa is dystrophic epidermolysis bullosa;
[5] The cell product of item [4], wherein said dystrophic epidermolysis bullosa is dominant dystrophic epidermolysis bullosa or recessive dystrophic epidermolysis bullosa;
[6] The cell product of any one of items [1] to [5], wherein said pluripotent stem cell is one having all of the following characteristics:

(i) having low or no telomerase activity;
(ii) capable of differentiating into any of tridermic cells;
(iii) showing no neoplastic proliferation; and

(iv) having self-renewal capacities;

[7] The cell product of any one of items [1] to [5], wherein said pluripotent stem cell is one having all of the following characteristics:

(i) SSEA-3 positive;
(ii) CD105 positive;
(iii) having low or no telomerase activity;
(iv) capable of differentiating into any of tridermic cells;
(v) showing no neoplastic proliferation; and
(vi) having self-renewal capacities;

[8] A skin cell differentiated from a SSEA-3-positive pluripotent stem cell derived from a mesenchymal tissue in a living body or a cultured mesenchymal cell;

[9] The skin cell of item [8], wherein said skin cell is a keratinocyte and/or a fibroblast;

[10] A cell product for treatment of a skin disease, comprising the skin cell of item [8] or [9];

[11] The cell product of item [10], wherein said skin disease is epidermolysis bullosa;

[12] A method for treating epidermolysis bullosa, comprising a step of administering an effective dose of the cell product of any one of items [1] to [6] to a patient in need thereof.

According to the present invention, Muse cells can be administered to a patient with epidermolysis bullosa via a blood vessel or the like, or directly to its skin site forming a blister or erosion and its periphery, to reconstruct and repair the damaged skin, thereby ameliorating or healing the skin symptoms. Thus, the cell product comprising Muse cells of the present invention can be used for treatment of epidermolysis bullosa.

Since Muse cells can efficiently migrate and engraft to a skin site forming a blister or erosion, and then spontaneously differentiate into epidermal cells at the engraftment site, they do not require differentiation induction into cells to be treated prior to transplantation. In addition, Muse cells are non-tumorigenic and excellent in safety. Furthermore, since Muse cells does not induce any immune rejection, treatment with allogenic preparations produced from donors is also possible. Therefore, Muse cells having the excellent characteristics as described above can provide easy and viable means for treatment of patients with epidermolysis bullosa.

According to the present invention, skin cells differentiated from Muse cells, such as keratinocytes or fibroblasts, can be administered to a site affected by a skin disease and its periphery of a patient with the skin disease such as epidermolysis bullosa to reconstruct and repair the damaged skin, thereby ameliorating or healing the skin symptoms. Thus, the cell product comprising skin cells differentiated from Muse cells of the present invention can be used for treatment of skin diseases such as epidermolysis bullosa.

BRIEF DESCRIPTION OF THE DRAWINGS

FIG. 1 shows photographs of wound sites of full-thickness wound model mice in each group (day 0, 3, 6, or 9 after administration).

FIG. 2 is a graph showing changes over time in the epithelization rate in wound sites of full-thickness wound model mice in each group.

FIG. 3, the photomicrographs shows the results of staining of human nuclei and human collagen type VII (COL7) in tissue sections of wound sites of full-thickness wound model mice in each group.

FIG. 4 shows the skin state of a COL17-knockout mouse after administration of Muse cells (middle) or HBSS (right). The left shows a mouse before administration.

FIG. 5 shows the results of electrophoresis for RT-PCR analysis for the expressions of human COL7 gene (A) and human COL17 gene (B) in a skin of a COL17-knockout mouse administered with Muse cells. Lane L corresponds to a marker for electrophoresis; and lanes 1 to 6 show the results for six COL17-knockout mice administered with Muse cells; lane 7 for a normal human keratinocyte (positive control); lane 8 for a normal B6 mouse (negative control); and lane 9 for water only.

FIG. 6 depicts photomicrographs showing the results of human COL7 staining in skin tissue sections from a COL17-knockout mouse administered with Muse cells.

FIG. 7 shows Muse cells differentiating into keratinocytes.

FIG. 8 shows the results of immunostaining for expression of keratinocyte markers in a keratinocyte differentiated from a Muse cell.

FIG. 9 shows the results of RT-PCR for expression of keratinocyte markers in a keratinocyte differentiated from a Muse cell.

FIG. 10 shows Muse cells differentiating into fibroblasts.

FIG. 11 shows the results of immunostaining for expression of fibroblast markers in a fibroblast differentiated from a Muse cell.

FIG. 12 shows the results of RT-PCR for expression of fibroblast markers in a fibroblast differentiated from a Muse cell.

DETAILED DESCRIPTION OF THE INVENTION

<1> Cell product Comprising Muse Cell

The present invention relates to a cell product for treatment of epidermolysis bullosa, the cell product comprising a SSEA-3-positive pluripotent stem cell (Muse cell). The treatment includes healing, ameliorating, and preventing relapse of the symptoms. The present invention will be described in detail below.

1. Indications

The cell product comprising a SSEA-3-positive pluripotent stem cell (Muse cell) of the present invention is used for treatment of epidermolysis bullosa.

As used herein, the term "epidermolysis bullosa" refers to a serious hereditary bullous skin disease where genetic abnormalities in adhesion structure control proteins in a skin basement membrane zone, for example, cause disruption of the adhesion functions between the epidermis and dermis, allowing epidermis to peel off at the basement membrane level with a slight external force in daily life and forming a blister, ulcer and/or erosion.

In the present invention, epidermolysis bullosa is divided, depending on where blister form, into three main types: epidermolysis bullosa simplex, junctional epidermolysis bullosa, and dystrophic epidermolysis bullosa (e.g., dominant dystrophic epidermolysis bullosa, and recessive dystrophic epidermolysis bullosa). Epidermolysis bullosa causes a blister or erosion due to slight external forces at sites that are susceptible to external forces, such as peripheral extremities and large joints.

2. Cell product

(1) Pluripotent Stem Cell (Muse Cell)

The pluripotent stem cell used in the cell product of the present invention is a cell that was found in human living body and named "Muse (Multilineage-differentiating Stress Enduring) cell" by Dezawa et al. It is known that Muse cells can be obtained from, for example, bone marrow fluid, adipose tissues (Ogura, F., et al., Stem Cells Dev., Nov 20, 2013 (Epub) (published on Jan 17, 2014)) and dermal connective tissues of skin, and are broadly present in tissues and connective tissues in organs. This cell also has both characteristics of pluripotent stem cell and mesenchymal stem cell and is identified as, for example, a cell positive for "SSEA-3 (Stage-specific embryonic antigen-3)," a cell surface marker, preferably as a double-positive cell that is positive for SSEA-3 and CD-105. Therefore, Muse cells or a cell population containing Muse cells can be isolated from tissues in a living body using, for example, expression of SSEA-3 only or a combination of SSEA-3 and CD-105 as an index. Methods for separation and identification of, and characteristics of Muse cell have been specifically disclosed in WO2011/007900. Muse cells can also selectively enriched by utilizing their high resistance to various external stresses and culturing them under various external stress conditions, such as under protease treatment, under hypoxic condition, under low-phosphate condition, in a low serum concentration, under undernutrition condition, under heat shock exposure, in the presence of toxic substance, in the presence of active oxygen, under mechanical stimulation, and under pressure treatment. As used herein, the pluripotent stem cells (Muse cells) or a cell population containing Muse cells prepared, as a cell product for treating epidermolysis bullosa, from a mesenchymal tissue in a living body or cultured mesenchymal tissues using SSEA-3 as an index may be simply referred to as "SSEA-3-positive cells." As used herein, the term "non-Muse cells" may refer to cells contained in a mesenchymal tissue in a living body or cultured mesenchymal cells and excluding "SSEA-3-positive cells."

Muse cells or a cell population containing Muse cells can be prepared from tissues (e.g., mesenchymal tissues) in a living body using cell surface markers, SSEA-3, or SSEA-3 and CD-105, as an index(es). As used herein, the term "living" body means mammal living body. In the present invention, the living body does not include fertilized egg and embryos in developmental stages before blastula stage, but includes embryos in developmental stages of blastula stage or later, including fetus and blastula. Examples of the mammal include, but not limited to, primates such as human and monkey; rodents such as mouse, rat, rabbit, and guinea pig; and cat, dog, sheep, pig, cattle, horse, donkey, goat, and ferret. The Muse cell to be used in the cell product of the present invention is definitively distinguished from embryonic stem cells (ES cells) and induced pluripotent stem (iPS) cells in that the Muse cell is directly isolated from a tissue in a living body with a marker. The term "mesenchymal tissue" refers to tissues such as bone, synovial membrane, fat, blood, bone marrow, skeletal muscle, dermis, ligament, tendon, dental pulp, umbilical cord, cord blood, and amnion, and tissues present in various organs. The Muse cells can be obtained from, for example, bone marrow, skin, adipose tissue, blood, dental pulp, umbilical cord, cord blood, and amnion. Preferably, a mesenchymal tissue in a living body is collected, and then Muse cells are prepared from the tissue and used. Alternatively, using the preparation method described above, the Muse cells may be prepared from cultured mesenchymal cells such as fibroblast and bone marrow mesenchymal stem cell.

The cell population containing Muse cells to be used in the cell product of the present invention can also be prepared by a method comprising stimulating a mesenchymal tissue in a living body or cultured mesenchymal cells with an external stress to selectively allow cells with the resistance to the external stress to grow and collecting the cells with an increased abundance ratio.

The external stress may be any one of or a combination of the followings: protease treatment, culturing under low oxygen concentration, culturing under low-phosphate condition, culturing under low serum concentration, culturing undernutrition condition, culturing under heat shock exposure, culturing at low temperatures, freezing treatment, culturing in the presence of toxic substances, culturing in the presence of active oxygen, culturing under mechanical stimulation, culturing under shaking, culturing under pressure treatment or physical shocks.

The protease treatment is preferably carried out for 0.5 to 36 hours in total to exert the external stress. The concentration of the protease may be that used when cells adhered to a culture vessel are peeled off, when cell aggregates are separated into single cells, or when single cells are collected from a tissue.

Preferably, the protease is serine protease, aspartic protease, cysteine protease, metalloprotease, glutamic protease or N-terminal threonine protease. More preferably, the protease is trypsin, collagenase or Dispase.

The Muse cell to be used in the cell product of the present invention may be autologous or allogeneic to a recipient to be transplanted with the cell.

As described above, Muse cells or a cell population containing Muse cells can be prepared from tissues in a living body, for example, by using SSEA-3-positivity or SSEA-3 and CD-105-double-positivity as an index. Human adult skin is known to comprise various types of stem cells and precursor cells. However, Muse cell is different from these cells. These stem cells and precursor cells include skin-derived precursor cell (SKP), neural crest stem cell (NCSC), melanoblast (MB), pericyte (PC), endothelial precursor cell (EP), and adipose-derived stem cell (ADSC). Muse cells can be prepared using "non-expression" of markers unique to these cells as an index. More specifically, Muse cells can be isolated using as an index non-expression of at least one, e.g., 2, 3, 4, 5, 6, 7, 8, 9, 10 or 11, of 11 markers selected from the group consisting of CD34 (a marker for EP and ADSC), CD117 (c-kit) (a marker for MB), CD146 (a marker for PC and ADSC), CD271 (NGFR) (a marker for NCSC), NG2 (a marker for PC), vWF factor (von Willebrand factor) (a marker for EP), Sox10 (a marker for NCSC), Snail (a marker for SKP), Slug (a marker for SKP), Tyrp1 (a marker for MB), and Dct (a marker for MB). Muse cells can be prepared by using as an index non-expression of, for example, but not limited to, CD117 and CD146; CD117, CD146, NG2, CD34, vWF and CD271; or the above-described 11 markers.

The Muse cell having the above-described characteristics and used in the cell product of the present invention also has at least one selected from the group consisting of the following characteristics:

(i) having low or no telomerase activity;
(ii) capable of differentiating into any of tridermic cells;
(iii) showing no neoplastic proliferation; and
(iv) having self-renewal capacities.

Preferably, the Muse cell to be used in the cell product of the present invention has all of the characteristics described above.

With respect to (i) above, the phrase "having low or no telomerase activity" means that the telomerase activity is low or undetectable when detected using, for example, TRAPEZE XL telomerase detection kit (Millipore). Having "low" telomerase activity means, for example, having a telomerase activity comparable to somatic human fibroblast, or having 1/5 or less telomerase activity, preferably 1/10 or less telomerase activity, as compared with that of HeLa cell.

With respect to (ii) above, the Muse cell is capable of differentiating into triploblastic cells (endodermal, mesodermal, and ectodermal cells) in vitro and in vivo. For example, the Muse cell can be differentiated into hepatocyte (including cells expressing hepatoblastoma or hepatocyte markers), neuron, skeletal muscle cell, smooth muscle cell, osteocyte, or adipocyte by in vitro inductive culturing. The Muse cell may also be capable of differentiating into triploblastic cells when it is transplanted in testis in vivo. Further, the Muse cell is capable of migrating and engrafting to injured organs (such as heart, skin, spinal cord, liver, and muscle) when transferred to a living body via intravenous injection and differentiating into cells corresponding to tissues.

With respect to (iii) above, the Muse cells are characterized by proliferating at a growth rate of about 1.3 days and proliferating from one cell in suspension culture to form embryoid body-like cell aggregates and arrest their proliferation after about 14 days when the aggregates reach a certain size. When these embryoid body-like cell aggregates are transferred to adherent culture, the cells restart proliferation and cells proliferated from the cell aggregates spread at a growth rate of about 1.3 days. Further, the cells are characterized in that, when transplanted into testis, they do not become cancerous for at least half a year.

With respect to (iv) above, the Muse cell has self-renewal (self-replication) capacities. The term "self-renewal" means that the followings can be observed: differentiation into three-germ layer cells from cells contained in first embryoid body-like cell aggregates obtained by culturing one Muse cell in a suspension culture; as well as formation of second next-generation embryoid body-like cell aggregates by again culturing one cell of the first embryoid body-like cell aggregates in a suspension culture; and further differentiation into three-germ layer cells and formation of third embryoid body-like cell aggregates in a suspension culture from the second embryoid body-like cell aggregates. Self-renewal may be repeated for one or more cycles.

(2) Preparation and Use of Cell Product Comprising Muse Cell

The cell product comprising Muse cells of the present invention is obtained by, but the method is not limited thereto, suspending the Muse cells or the cell population containing the Muse cells obtained in (1) above in a physiological saline or a suitable buffer solution (e.g., phosphate buffered saline). In this case, if only small numbers of Muse cells are isolated from an autologous or allogeneic tissue, these cells may be cultured before cell transplantation until the determined number of cells is attained. As previously reported (WO2011/007900), since Muse cells do not become tumorigenic, even if cells collected from a living tissue are contained while remaining undifferentiated, they have low possibility of converting to malignant cells and thus are safe. The collected Muse cells can be cultured in any normal growth medium (e.g., alpha-minimum essential medium (a-MEM) supplemented with 10% calf serum). More specifically, with reference to the above-described WO2011/007900, Muse cells can be cultured and proliferated using an appropriately selected culture medium, additives (e.g., antibiotics, and serum) and the like, to prepare a solution containing Muse cells at the determined concentration. When the cell product comprising Muse cells of the present invention is administered to a human subject, bone marrow fluid can be collected from a human ilium. Then, for example, bone marrow mesenchymal stem cells can be cultured as adherent cells obtained from the bone marrow fluid and proliferated until they reach the cell amount where a therapeutically effective amount of Muse cells is obtained. Thereafter, Muse cells can be isolated using an antigenic marker SSEA-3 as an index, and these autologous or allogeneic Muse cells can be prepared into a cell product. Alternatively, for example, bone marrow mesenchymal stem cells obtained from the bone marrow fluid can be cultured under external stress conditions to proliferate and enrich Muse cells until they reach a therapeutically effective amount. Then, these autologous or allogeneic Muse cells can be prepared into a cell product.

When the Muse cells are used in the cell product, the cell product may contain dimethyl sulfoxide (DMSO), serum albumin and the like for protection of the cells and antibiotics and the like for prevention of contamination and proliferation of bacteria. The cell product may further contain other pharmaceutically acceptable components (e.g., carrier, excipient, disintegrant, buffer agent, emulsifier, suspending agent, soothing agent, stabilizer, preservative, antiseptic, physiological saline). These agents and drugs can be added to the cell product at appropriate concentrations by the skilled person. Thus, Muse cells can also be used as a pharmaceutical composition containing various additives.

The number of Muse cells contained in the cell product prepared above can be appropriately adjusted to achieve desired effects in treatment of epidermolysis bullosa, in consideration of, for example, sex, age, and weight of the subject, condition of the affected area, and condition of the cell to be used. Individuals as the subject includes, but not limited to, mammals such as human. The cell product comprising Muse cells of the present invention may be administered multiple times at appropriate intervals (e.g., twice a day, once a day, twice a week, once a week, once every two weeks, once a month, once every two months, once every three months, or once every six months) as appropriate until a desired therapeutic effect is achieved. Thus, depending on the state of the subject, preferred therapeutically effective amount is, for example, $1 \times 10^3$ to $1 \times 10^{10}$ cells/individual/dose in 1 to 10 doses per year. Examples of total dosage for an individual include, but not limited to, $1 \times 10^3$ to $1 \times 10^{11}$ cells, preferably $1 \times 10^4$ to $1 \times 10^{10}$ cells, more preferably $1 \times 10^5$ to $1 \times 10^9$ cells.

The Muse cell to be used in the cell product of the present invention is characterized by migrating and engrafting to damaged skin from epidermolysis bullosa. Thus, the cell product may be administered to any site by any method, e.g., locally to the affected area or intravenously to a vein.

The cell product comprising Muse cells of the present invention can repair and regenerate a damaged skin of a patient with epidermolysis bullosa. Repair and regeneration of the damaged skin can be determined by, for example, recovery and/or increase of expression of collagen proteins such as COL7 and COL17. Thus, the cell product comprising Muse cells of the present invention has an effect of recovering and/or increasing expression of collagen proteins.

<2> Skin Cell Differentiated from Muse Cell and Cell product Comprising the Skin Cell

In the present invention, skin cells differentiated from Muse cells, such as keratinocytes and/or fibroblasts, can be used as a cell product.

Keratinocytes refer to a cell that produces keratin and also referred to as epidermal cell or keratinized cell. Muse cells can be differentiated into keratinocytes, for example, by culturing Muse cells in a culture medium containing keratinocyte growth factor (KGF) and epidermal growth factor (EGF), preferably by culturing Muse cells in a culture medium containing KGF and EGF followed by culturing in a culture medium containing KGF, EGF, hepatocyte growth factor (HGF), and insulin-like growth factor 2 (IGF2). Preferred and exemplified concentrations range from 5 to 20 ng/ml for KGF, from 20 to 40 ng/ml for EGF, and from 40 to 80 ng/ml for IGF2. Preferred and exemplified culturing period ranges from 7 to 28 days.

Fibroblasts refer to a cell that produces dermis components such as collagen and elastin. Muse cells can be differentiated into fibroblasts, for example, by culturing Muse cells in a culture medium containing transforming growth factor-$\beta$2 (TGF-$\beta$2) and ascorbic acid (AA), preferably by culturing Muse cells in a culture medium containing TGF-$\beta$2 and AA followed by culturing in a culture medium containing AA. Preferred and exemplified concentrations range from 30 to 60 $\mu$g/ml for TGF-$\beta$2, and from 20 to 80 mmol/l for AA. Preferred and exemplified culturing period ranges from 7 to 28 days.

The cell product comprising skin cells differentiated from Muse cells, such as keratinocytes and/or fibroblasts, can be used in treatment not only for epidermolysis bullosa but also for skin diseases in general that are curable by skin cell replacement therapy.

When the cell product comprising skin cells differentiated from Muse cells is used, the cell product may contain dimethyl sulfoxide (DMSO), serum albumin and the like for protection of the cells and antibiotics and the like for prevention of contamination and proliferation of bacteria. The cell product may further contain other pharmaceutically acceptable components (e.g., carrier, excipient, disintegrant, buffer agent, emulsifier, suspending agent, soothing agent, stabilizer, preservative, antiseptic, physiological saline).

The dose of the cell product can be appropriately adjusted to achieve desired effects in treatment of a skin disease, in consideration of, for example, sex, age, and weight of the subject, condition of the affected area, and condition of the cell to be used. Individuals as the subject includes, but not limited to, mammals such as human. The cell product may be administered multiple times at appropriate intervals (e.g., twice a day, once a day, twice a week, once a week, once every two weeks, once a month, once every two months, once every three months, or once every six months) as appropriate until a desired therapeutic effect is achieved. Thus, depending on the state of the subject, preferred therapeutically effective amount is, for example, $1 \times 10^3$ to $1 \times 10^{10}$ cells/individual/dose in 1 to 10 doses per year. Examples of total dosage for an individual include, but not limited to, $1 \times 10^3$ to $1 \times 10^{11}$ cells, preferably $1 \times 10^4$ to $1 \times 10^{10}$ cells, more preferably $1 \times 10^5$ to $1 \times 10^9$ cells. The cell product may be administered by any method, and local administration to a site affected by a skin disease or its periphery is preferred. The skin cell may be formed into a sheet and applied to the affected site.

The present invention will be described in more detail with reference to examples below, but is not limited to the examples in any way.

EXAMPLES

Preparation of Human Muse Cell

Muse cells were obtained according to the method described in WO2011/007900 on isolation and identification of human Muse cells. The Muse cells were obtained by expansive enrichment culture of mesenchymal stem cells under stress conditions. A commercially available MSC was purchased and used as the MSC group.

Example 1. Evaluation with Full-thickness Wound Model Mouse

A full-thickness wound was made on the back of an adult C57BL/6 mouse, and the mouse was used as a full-thickness wound model. Within 30 minutes after full-thickness wounding, the above-prepared Muse cells ($3 \times 10^5$ per mouse or $3 \times 10^4$ per mouse), MSCs ($3 \times 10^5$ per mouse), or 200 $\mu$l of HBSS was injected into the tail vein, and then the therapeutic effect was investigated. The epithelization rate was calculated as below.

The back skins at the time of wounding and at day 3, 6, 9, and 11 after wounding were photographed with a digital camera together with a ruler, and then the skin ulcer areas (mm$^2$) were determined using Image J software (version 1.50i). Using the area at the time of wounding as a reference value, the percent area reduction was calculated.

$$\text{Epithelization rate (\%)} = \frac{\text{Area at the time of wounding (mm}^2) - \text{Ulcer area at each day (mm}^2)}{\text{Area at the time of wounding (mm}^2)} \times 1\,0\,0$$

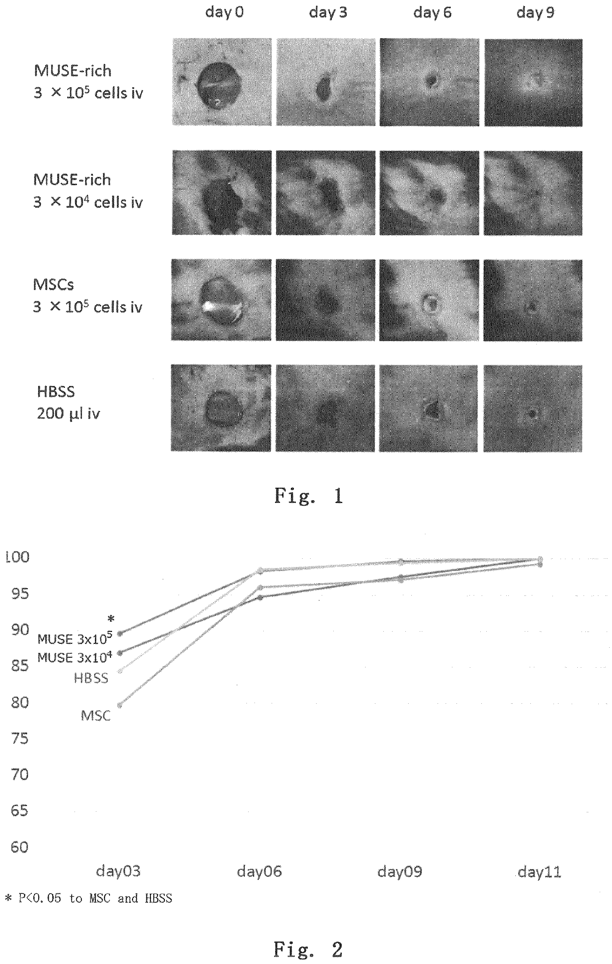

The results are shown in FIGS. 1 and 2. In all groups, the wounds were healed with time. At day 3, the Muse cell ($3 \times 10^5$ per mouse)-treated group showed a significantly faster healing and higher epithelization rate than the MSC-treated group and the HBSS-treated group, demonstrating that administration of Muse cells is effective in treatment of epidermolysis bullosa.

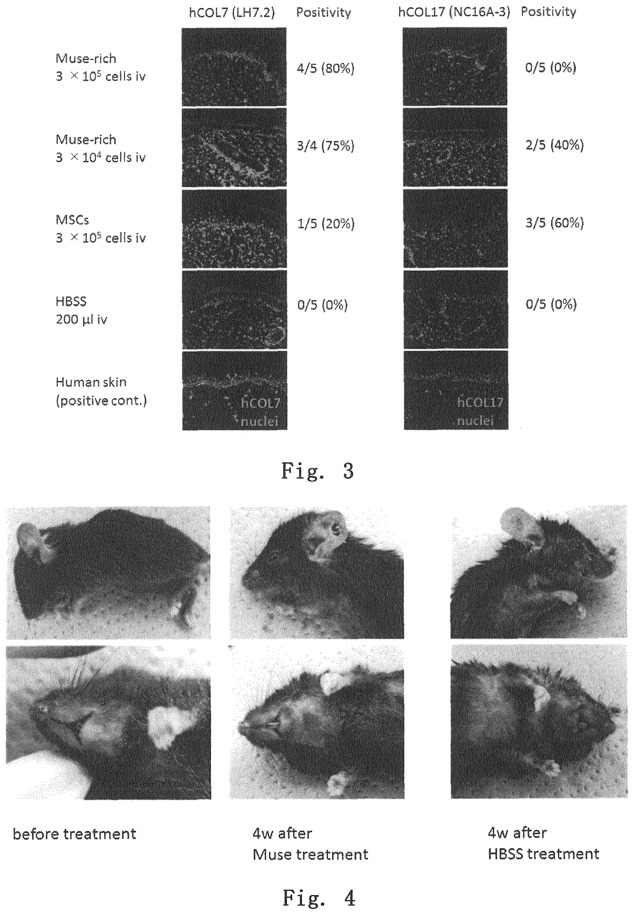

At day 14, removing the skin tissue at the wound site, it was prepared into a section and then stained for nucleus and human COL7. As shown in FIG. 3, it was found that the Muse cell-treated group showed the presence of human COL7 in epidermis and dermis, demonstrating that the administered Muse cells migrated to the skin and produced the molecule that is needed for adhesion between epidermis and dermis.

Example 2. Evaluation with COL17-knockout Epidermolysis Bullosa Model Mouse

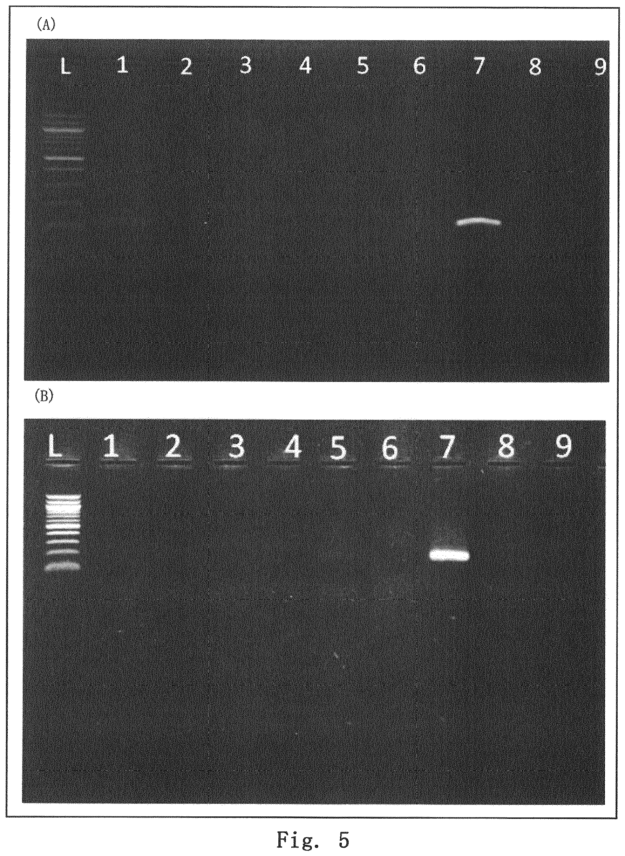

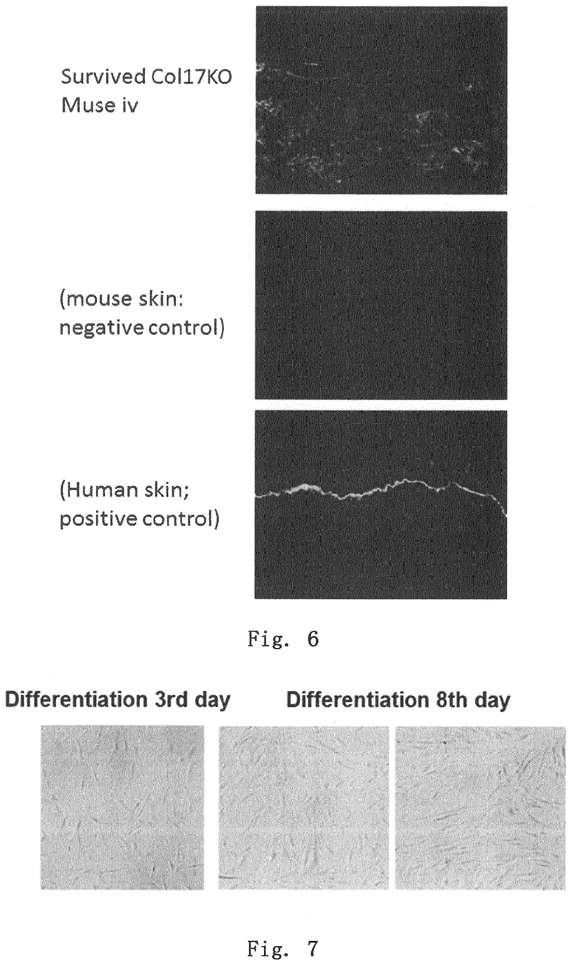

Using a 3 to 4-week COL17 gene-knockout mouse (see Nat Med. 2007 Mar; 13 (3): 378-83.), the epidermis was chafed to form a blister. Then, within 30 minutes after the blister formation, Muse cells ($3 \times 10^5$ per mouse) were injected into the tail vein. Observing the skin state after one month, as shown in FIG. 4, the control mouse treated with HBSS had a poor hair coat, showing extensive formation of wounds and mucosal erosion, while the mouse treated with Muse cells gave mild results for both hair coat and wound formation. In addition, the skin tissue one month after the administration was removed, and RNA was extracted from the skin tissue. RT-PCR was performed to examine the expression of human-derived COL7 and COL17 genes. The results are shown in FIG. 5. As can be seen from the results, the Muse cell-administered mice showed the presence of human COL7 and human COL17, and thus the administered Muse cells provided the adhesion factors. The expression of human COL7 was also detected at protein level (FIG. 6).

Example 3. Differentiation of Muse Cells into Keratinocytes

Differentiation of Muse cells into keratinocytes was made by culturing them according to the following procedure:

Day 0: plating Muse cells;
Day 1: culturing them in a DMEM low glucose medium supplemented with 10% FBS, KGF (10 ng/ml), and EGF (20 to 30 ng/ml) for three days; and
Day 4: culturing them in a DMEM low glucose medium supplemented with 10% FBS, KGF (10 ng/ml), EGF (20 to 30 ng/ml), HGF (10 ng/ml), and IGF2 (60 ng/ml) for 8 to 14 days with the culture medium exchanged every other day.

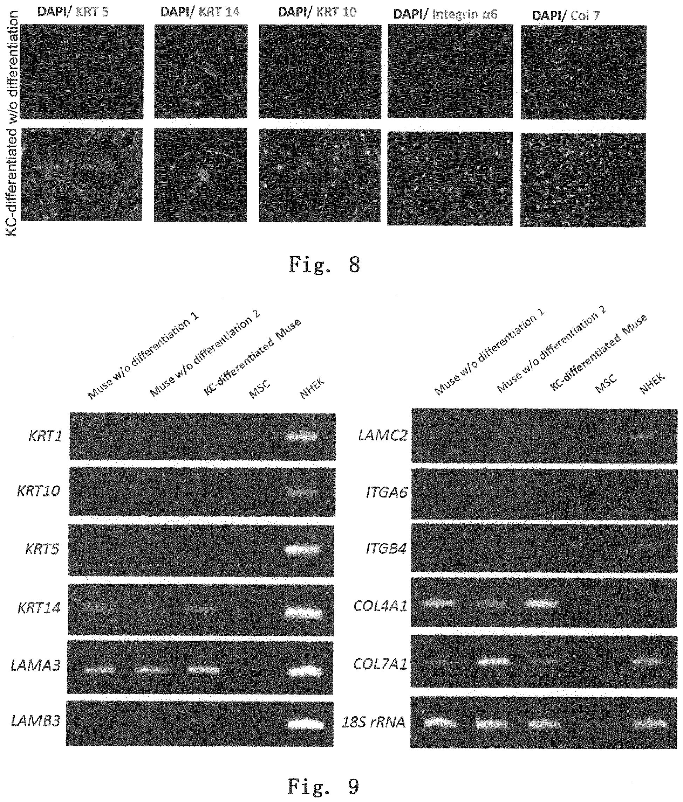

The results are shown in FIGS. 7 to 9. As shown in FIG. 7, cells at day 8 of differentiation showed keratinocyte-like morphology. In addition, as shown in FIGS. 8 and 9, differentiated cells expressed keratinocyte markers at protein and mRNA levels.

Example 4. Differentiation of Muse Cells into Fibroblasts

Differentiation of Muse cells into fibroblasts was made by culturing them according to the following procedure:

Day 0: plating Muse cells;
Day 1: culturing them in 10 ml of DMEM low glucose medium supplemented with 2 μl of TGF-β2 (50 μg/ml), 60 μl of AA (50 mM), and 100 μl of ITS-A (Insulin, Transferrin, Sodium selenite) for 6 days with the culture medium exchanged every other day:

Day 7: culturing them in a DMEM low glucose medium supplemented with 20% FBS and 60 μl of AA (50 mM) for 10 days with the culture medium exchanged every other day: and
Day 17: culturing them in a DMEM low glucose medium supplemented with 10% FBS.

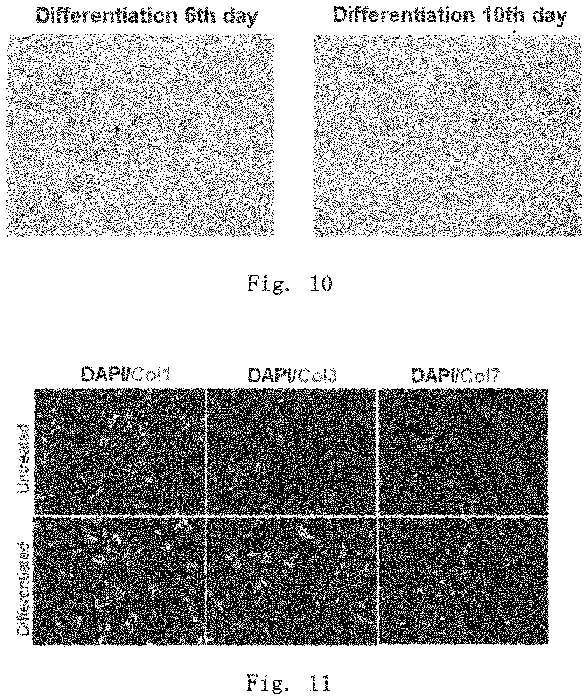

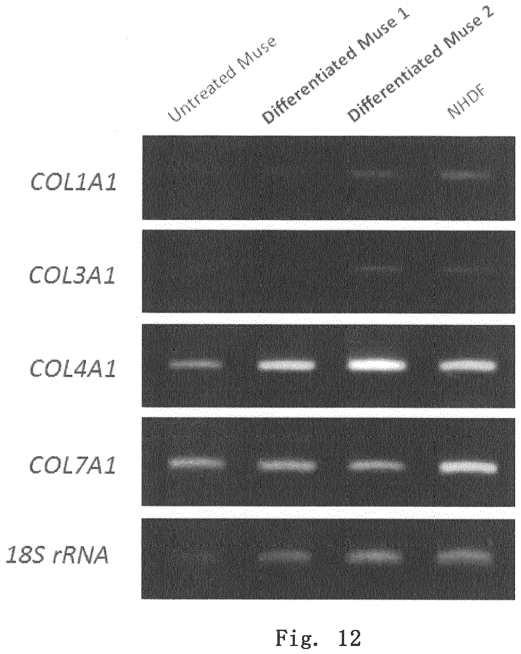

The results are shown in FIGS. 10 to 12. As shown in FIG. 10, cells at day 10 of differentiation showed fibroblast-like morphology. In addition, as shown in FIGS. 11 and 12, differentiated cells expressed fibroblast markers at protein and mRNA levels.

Industrial Applicability

The cell product of the present invention can be administered to a patient with epidermolysis bullosa to reconstruct and repair the damaged skin, ameliorating or healing the skin symptoms, and thus can be applied to treatment of epidermolysis bullosa.

**Claims**

1. A cell product for treatment of epidermolysis bullosa, comprising a SSEA-3-positive pluripotent stem cell derived from a mesenchymal tissue in a living body or a cultured mesenchymal cell.

2. The cell product of claim 1, wherein said epidermolysis bullosa is epidermolysis bullosa simplex.

3. The cell product of claim 1, wherein said epidermolysis bullosa is junctional epidermolysis bullosa.

4. The cell product of claim 1, wherein said epidermolysis bullosa is dystrophic epidermolysis bullosa.

5. The cell product of claim 4, wherein said dystrophic epidermolysis bullosa is dominant dystrophic epidermolysis bullosa or recessive dystrophic epidermolysis bullosa.

6. The cell product of any one of claims 1 to 5, wherein said pluripotent stem cell has all of the following characteristics:

    (i) having low or no telomerase activity;
    (ii) capable of differentiating into any of tridermic cells;
    (iii) showing no neoplastic proliferation; and
    (iv) having self-renewal capacities.

7. The cell product of any one of claims 1 to 5, wherein said pluripotent stem cell has all of the following characteristics:

    (i) SSEA-3 positive;
    (ii) CD105 positive;
    (iii) having low or no telomerase activity;
    (iv) capable of differentiating into any of tridermic cells;
    (v) showing no neoplastic proliferation; and
    (vi) having self-renewal capacities.

8. A skin cell differentiated from a SSEA-3-positive pluripotent stem cell derived from a mesenchymal tissue in a living body or a cultured mesenchymal cell.

9. The skin cell of claim 8, wherein said skin cell is a keratinocyte and/or a fibroblast.

10. A cell product for treatment of a skin disease, comprising the skin cell of claim 8 or 9.

11. The cell product of claim 10, wherein said skin disease is epidermolysis bullosa.

Fig. 1

* P<0.05 to MSC and HBSS

Fig. 2

|  | hCOL7 (LH7.2) | Positivity | hCOL17 (NC16A-3) | Positivity |
|---|---|---|---|---|
| Muse-rich<br>3 × 10⁵ cells iv | | 4/5 (80%) | | 0/5 (0%) |
| Muse-rich<br>3 × 10⁴ cells iv | | 3/4 (75%) | | 2/5 (40%) |
| MSCs<br>3 × 10⁵ cells iv | | 1/5 (20%) | | 3/5 (60%) |
| HBSS<br>200 μl iv | | 0/5 (0%) | | 0/5 (0%) |
| Human skin<br>(positive cont.) | | | | |

Fig. 3

before treatment          4w after                4w after
                          Muse treatment          HBSS treatment

Fig. 4

Fig. 5

Survived Col17KO
Muse iv

(mouse skin:
negative control)

(Human skin;
positive control)

Fig. 6

Differentiation 3rd day    Differentiation 8th day

Fig. 7

Fig. 8

Fig. 9

**Differentiation 6th day**　　**Differentiation 10th day**

Fig. 10

Fig. 11

Fig. 12

## INTERNATIONAL SEARCH REPORT

| International application No. |
| --- |
| PCT/JP2018/023352 |

### A. CLASSIFICATION OF SUBJECT MATTER

Int.Cl. A61K35/545(2015.01)i, A61K35/28(2015.01)i, A61K35/33(2015.01)i,
A61K35/36(2015.01)i, A61P17/00(2006.01)i, A61P17/02(2006.01)i,
C12N5/071(2010.01)i, C12N5/0775(2010.01)n

According to International Patent Classification (IPC) or to both national classification and IPC

### B. FIELDS SEARCHED

Minimum documentation searched (classification system followed by classification symbols)

Int.Cl. A61K35/545, A61K35/28, A61K35/33, A61K35/36, A61P17/00,
A61P17/02, C12N5/071, C12N5/0775

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

JSTPlus/JMEDPlus/JST7580 (JDreamIII), CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X<br>Y | 玉井克人，表皮水疱症に対する間葉系幹細胞移植再生医療の実用化研究平成24-26年度総合研究報告書，表皮水疱症に対する間葉系幹細胞移植再生医療の実用化研究平成24-26年度総合研究報告書, May 2015, pp. 1-16, page 4 "2013 1) Clinical research on mesenchymal stem cell transplantation", page 5 "2014 1) Clinical research on mesenchymal stem cell transplantation", page 3, right column to page 4, left column "2012", page 4, right column to page 5, left column "2) Validity evaluation of systemic mesenchymal stem cell transplantation", page 6, left column, lines 6-16, page 6, right column, lines 25-30, non-official translation (TAMAI, Katsuto, "2012-2014 comprehensive research report on practical use of mesenchymal stem cell transplantation regenerative medicine against epidermolysis bullosa") | 1,4-7<br>1-7 |

☒ Further documents are listed in the continuation of Box C.  ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 07 September 2018 (07.09.2018) | 18 September 2018 (18.09.2018) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2018/023352 |

C (Continuation). DOCUMENTS CONSIDERED TO BE RELEVANT

| Category* | Citation of document, with indication, where appropriate, of the relevant passages | Relevant to claim No. |
|---|---|---|
| X | PETROF, G., et al., "Potential of Systemic Allogenic Mesenchymal Stromal Cell Therapy for Children with Recessive Dystrophic Epidermolysis Bullosa", Journal of Investigative Dermatology, 2015, vol. 135, pp. 2319-2321, page 2319, middle column, lines 3-6, page 2319, right column, line 31 to page 2320, right column, line 35 | 1,4-7 |
| X | TOLAR, J. et al., "MATOPOIETIC AND MESENCHYMAL CELL TRANSPLANTATION AFTER MYELOABLATIVE AND NON-MYELOABLATIVE CONDITIONING FOR RECESSIVE DYSTROPHIC AND JUCTIONALEPIDERMOLYSIS BULOSA (RDEB, JEB)", Wound Rep. Reg., 2012, vol. 20, p. A67, entire text | 1,3-7 |
| X Y | JP 2016-56109 A (THE UNIVERSITY OF TOKYO) 21 April 2016, examples 4-5 & US 2017/0258844 A1, examples 4-5 & WO 2016/035419 A1 & EP 3189844 A1 & AU 2015310286 A & CA 2959957 A & KR 10-2017-0055477 A & CN 107073041 A | 8-9 1-11 |
| X | JP 2016-516394 A (CLIO, INC.) 09 June 2016, claims 1-5, 12, examples 2-3 & WO 2014/163206 A1, claims 1-5, 12, examples 2-3 | 8,10 |
| X Y | SHIN, K. C., et al., "The Use of Cultured Allogenic Keratinocyte Grafting in a Patient with Epidermolysis Bullosa Simplex", Ann. Dermatol., 2011, vol. 23, suppl. 3, pp. S393-S397, abstract, p. S393-S395 "CASE REPORT" | 8-11 8-11 |
| X Y | ZARE, S. et al., "Application of Allogeneic Fibroblast Cells in Cellular Therapy of Recessive Dystrophic Epidermolysis Bullosa", J. Skin Stem Cell, 2015, vol. 2, no. 3, e30787 (6pages), page 3, right column, lines 5-15, fig. 2, 3 | 8-11 8-11 |
| Y | DEZAWA, M., "Muse Cells Provide the Pluripotency of Mesenchymal Stem Cells; Direct Contribution of Muse Cells to Tissue Regeneration", Cell Transplantation, 2016, vol. 25, pp. 849-861, page 857, left column, lines 5-9, page 857, right column to page 858, right column "MUSE CELLS MAY RESOLVE THE PROBLEMS OF MSCs" | 1-11 |
| Y | WO 2012/133948 A1 (CLIO, INC.) 04 October 2012, claims 1-10, examples 1-3 & US 2012/0244129 A1, claim 26, paragraphs [0177]-[0179], examples 5-7 & JP 2014-133703 A & US 2016/0369232 A1 & CN 103442724 A | 1-7 |

Form PCT/ISA/210 (continuation of second sheet) (January 2015)

**INTERNATIONAL SEARCH REPORT**

| International application No. |
| --- |
| PCT/JP2018/023352 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | PAUNESCU, V. et al., "In vitro differentiation of Human mesenchymal stem cells to epithelial lineage", J. Cell. Mol. Med., 2007, vol. 11, no. 3, pp. 502-508, page 503, right column "Differentiation of MSCs to epithelial-like cells" | 8-11 |
| Y | ITOH, M. et al., "Differentiation of human induced pluripotent stem cells into dermal fibroblast", Journal of Investigative Dermatology, 2012, vol. 132, suppl. 1, S140 abstract no. 817, entire text | 8-11 |
| P,X | FUJITA, Y. et al., "CL2020, a human multilineage-differentiating stress enduring cells-rich product, has a potential to treat dystrophic epidermolysis bullosa", Journal of Investigative Dermatology, October 2017, vol. 137, no. 10, suppl. 2, p. S224, abstract, no. 186, entire text | 1-7 |
| P,X | YAMAUCHI, T. et al., "The Potential of Muse cells for Regenerative Medicine of Skin: Procedures to Reconstitute Skin with Muse Cell-Derived Keratinocytes, Fibroblasts, and Melanocytes", Journal of Investigative Dermatology, 14 October 2017, vol. 137, no. 12, pp. 2639-2642, fig. 1 | 8-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5185443 B
- WO 2011007900 A

### Non-patent literature cited in the description

- **TAMAI K.** *J Natl Inst Public Health,* 2011, vol. 60, 118-124
- **CHINO T et al.** *Am J Pathol,* 2008, vol. 173, 803-814
- **TAMAI K et al.** *Proc Natl Acad Sci USA,* 2011, vol. 108, 6609-6614
- **WAGNER JE et al.** *N Engl J Med,* 2010, vol. 363, 629-639
- **CONGET P et al.** *Cytotherapy,* 2010, vol. 12, 429-431
- **PETROF G et al.** *J Invest Dermatol,* 2015, vol. 135, 2319-2321
- **EL-DAROUTI M et al.** *Dermatol Ther,* 2016, vol. 29, 96-100
- **KURODA Y et al.** *Proc Natl Acad Sci USA,* 2010, vol. 107, 8639-8643
- **WAKAO S et al.** *Proc Natl Acad Sci USA,* 2011, vol. 108, 9875-9880
- **KURODA Y et al.** *Nat Protc,* 2013, vol. 8, 1391-1415
- **OGURA, F. et al.** *Stem Cells Dev.,* 20 November 2013
- *Nat Med.,* March 2007, vol. 13 (3), 378-83